# EUROPÄISCHE PATENTSCHRIFT

(11) **EP 2 313 076 B1**
(45) Veröffentlichungstag und Bekanntmachung des Hinweises auf die Patenterteilung: **09.07.2014**
(21) Anmeldenummer: 09780004.9
(22) Anmeldetag: 29.06.2009
(51) Int. Cl.: A61K 8/85, A61Q 17/04, A61Q 19/10, A61Q 5/00, A61Q 5/02, A61Q 5/12, A61K 8/81

(54) **RINSE-OFF-PRODUKTE MIT BENEFIT-WIRKUNG AUF DIE HAUT**
RINSE-OFF PRODUCTS HAVING A BENEFICIAL EFFECT ON THE SKIN
PRODUITS À RINCER AYANT UN EFFET BÉNÉFIQUE SUR LA PEAU

(30) Priorität: 09.07.2008 DE 102008032179; 29.12.2008 DE 102008063307; 29.12.2008 DE 102008063288
(43) Veröffentlichungstag der Anmeldung: 27.04.2011
(73) Patentinhaber: Henkel AG & Co. KGaA, 40589 Düsseldorf (DE)
(72) Erfinder: BARRELEIRO, Paula, 40593 Düsseldorf (DE); ZIGANKE, Kerstin, 40721 Hilden (DE); JANßEN, Frank, 41470 Neuss (DE); COX, Bruce, Scottsdale AZ 85259 (US)
(86) Internationale Anmeldenummer: PCT/EP2009/058109
(87) Internationale Veröffentlichungsnummer: WO 2010/003843

(56) Entgegenhaltungen:
- EP-A- 0 573 229
- EP-A- 1 813 266
- WO-A-00/09081
- DE-A1-102008 052 053
- US-A1- 2007 264 216
- US-B1- 7 025 952

## Beschreibung

Die vorliegende Erfindung betrifft die Verwendung von Tensid-haltigen Rinse-off-Produkten, die mindestens einen Filmbildner und mindestens eine Benefit-Substanz, ausgewählt aus UV-Filtersubstanzen und Bräunungsmitteln, enthalten, zur Reinigung der Haut und gleichzeitigen Bewirkung eines Benefit-Effektes auf der Haut.

Sogenannte Rinse-off-Produkte dienen in der Regel der Reinigung der Haut. Primäres Ziel dieser Produkte ist es in diesem Sinne, Substanzen von der Haut zu entfernen. Zu diesem Zweck enthalten sie in der Regel insbesondere hohe Mengen an Tensiden, um die Aufnahme und Solubilisierung bzw. Emulgierung der von der Haut zu entfernenden Verunreinigungen und damit letztlich deren Entfernung infolge des Abspülens der entsprechend behandelten Haut mit Wasser zu ermöglichen. Dagegen werden sogenannte Leave-on-Produkte dazu verwendet, um die Haut mit Benefit-Substanzen, also mit Substanzen, die der Haut eine positive Eigenschaft verleihen, auszustatten. Bei dieser Wirkung kann es sich beispielsweise um eine hautpflegende, -straffende, - verjüngende Eigenschaft oder beispielsweise um die Verleihung von Schutz gegenüber Einwirkungen von außen, insbesondere etwa gegenüber Sonnenlicht, handeln.

Um die Haut einerseits zu reinigen, andererseits nachhaltig mit Benefit-Substanzen zu versorgen, ist daher in der Regel die Anwendung von mindestens zwei Behandlungsschritten erforderlich, nämlich die Behandlung mit einem reinigenden Rinse-Off-Produkt und der anschließenden Behandlung mit einem pflegenden Leave-On-Produkt.

Es ist jedoch wünschenswert, Produkte zur Verfügung zu haben, die die reinigenden Eigenschaften von Rinse-Off-Produkten und die pflegenden Eigenschaften von Leave-On-Produkten miteinander vereinen, dergestalt, dass bei der Auftragung und kurz darauf folgenden Entfernung eines so ausgestalteten Produktes durch Spülen mit Wasser sowohl eine Reinigung der Haut als auch eine nachhaltige Ausstattung mit Benefit-Substanzen erfolgen kann. Auf diese Weise könnte auf die Anwendung mehrerer Produkte in aufeinander folgenden Schritten verzichtet werden. Hinzu kommt, dass heute herkömmlicherweise verwendete Leave-On-Produkte, wie etwa Sonnencremes, aufgrund ihrer sensorischen Eigenschaften von vielen Verbrauchern als unangenehm empfunden werden. Es wäre auch aus diesem Grunde wünschenswert, die gewünschte Verleihung von Sonnenschutz, zu erzielen, ohne auf die Verwendung der heute gebräuchlichen Formulierungen angewiesen zu sein. In dieser Hinsicht sind im Stand der Technik bereits Rinse-Off-Produkte beschrieben, die dazu in der Lage sind, eine Ausstattung der Haut mit Benefit-Substanzen zu bewirken.

So werden in dem Patent EP1082101 B1 Zusammensetzungen offenbart, die Tensid, Wasser, ein geringe Menge kationisches Polymer als Ablagerungshilfe sowie in einer Matrix eingekapseltes Sonnenschutz-mittel enthalten. In dem Patent EP714283 B1 werden O/W-Emulsionen offenbart, die Wasser, einen kationischen Emulgator, einen alkoxylierten Ether, die Ablagerung unterstützende Polymere sowie Salicylsäure oder eine Substanz mit einem ähnlichen Löslichkeitsparameter wie Salicylsäure enthalten. In dem Patent EP1501873 B1 wird eine Zusammensetzung offenbart, die kationische Polygalactomannane oder Derivate davon enthält.

In dem Patent EP744935 B2 werden Zusammensetzungen offenbart, die Tensid, Wasser, ein kationisches Copolymer als Ablagerungshilfe sowie ein flüchtiges, unlösliches Silikonöl enthält. Auch in den Patentanmeldungen WO2005/068594 und WO2006/083843 werden Zusammensetzungen offenbart, die kationische Polymere als Ablagerungshilfe enthalten.

US 7025925 B1 offenbart Tensid-haltige Zusammensetzungen zur gleichzeitigen Reinigung der Haut und Verleihung von Sonnenschutz, die einen Filmbildner und UV-Filtersubstanzen enthalten. Überraschenderweise wurde nun erfindungsgemäß gefunden, dass tensidhaltige Zusammensetzungen, die Filmbildner enthalten, sehr gut zur Lösung der erfindungsgemäßen Aufgabe geeignet sind, indem sie trotz kurzer Einwirkzeit dazu in der Lage sind, eine effektive Ausstattung der Haut mit Benefit-Substanzen wie Bräunungsmitteln und/oder UV-Filtersubstanzen zu bewirken. Der Effekt kann überraschenderweise hierbei auch bei hohem Tensid- und/oder Öl-Gehalt der Zusammensetzung bewirkt werden.

Ein erster Gegenstand der vorliegenden Erfindung ist daher die Verwendung von tensidhaltigen Zusammensetzungen, die mindestens einen Filmbildner und mindestens eine Benefit-Substanz enthalten, zur Reinigung der Haut und gleichzeitigen Bewirkung eines Benefit-Effektes auf der Haut.

Erfindungsgemäße tensidhaltige Zusammensetzungen umfassen 2 - 30 Gew.-%, bevorzugt 3 - 20 Gew.-%, insbesondere 5 - 15 Gew.-%, an Filmbildnern sowie 0,1 - 20 Gew.-%, vorzugsweise 0,5 -10 Gew.-%, an Benefit-Substanzen.

Ein weiterer Gegenstand der vorliegenden Erfindung sind daher tensidhaltige Zusammensetzungen, enthaltend:
a) 2 - 30 Gew.-%, insbesondere 3 - 20 Gew.-%, vorzugsweise 5 - 15 Gew.-%, besonders bevorzugt 7,5 - 15 Gew.-%, Filmbildner,
b) 0,1 - 20 Gew.-%, vorzugsweise 0,5 - 10 Gew.-%, mindestens einer Benefit-Substanz, welche ausgewählt ist aus der Gruppe, bestehend aus UV-Filtersubstanzen und Bräunungsmitteln,
   wobei ein nichtionischer Filmbildner, der ein Copolymer aus N-Vinylpyrrolidon- und C₁₀₋₂₀-Alkylen-Einheiten ist, und ein nichtionischer Filmbildner, der ein Copolymer aus N-Vinylpyrrolidon-, Methacrylamid- und N-Vinyl-Imidazol-Einheiten ist, enthalten sind.

Erfindungsgemäße Zusammensetzungen enthalten weiterhin vorzugsweise mindestens einen Fettstoff, insbesondere in einer Menge von 0,1 - 50 Gew.-%, vorzugsweise in einer Menge von 2 - 45 Gew.-%, besonders bevorzugt in einer Menge von 5 - 40 Gew.-%, vor allem in einer Menge von 10 - 35, insbesondere in einer Menge von 15 - 35 Gew.-%.

Die erfindungsgemäße Verwendung als Rinse-Off-Produkt bzw. zur Reinigung der Haut erfolgt erfindungsgemäß vorzugsweise dadurch, dass eine erfindungsgemäße Zusammensetzung, die Filmbildner und mindestens eine Benefit-Substanz, ausgewählt aus UV-Filtersubstanzen und Bräunungsmitteln, enthält, auf die Haut aufgetragen wird und nach einer Einwirkzeit von maximal 5 Minuten, vorzugsweise von maximal 3 Minuten, insbesondere von maximal einer Minute, 30 Sekunden, 15 Sekunden oder 10 Sekunden wieder abgespült wird, vorzugsweise unter Verwendung von Wasser.

Die erfindungsgemäße Verwendung der erfindungsgemäßen tensidhaltigen Mittel erfolgt, um neben der Reinigung gleichzeitig einen Benefit-Effekt auf der Haut zu bewirken, wobei es sich bei dem Benefit-Effekt um die Ausstattung der Haut mit einem Bräunungsmittel oder um die Verleihung von Schutz gegenüber Sonnenlicht (Sonnenschutz) handelt.

Bei Filmbildnern handelt es sich um Substanzen, die in einem Lösungsmittel gelöst - oder gegebenenfalls dispergiert - auf Haut, Haar oder Nägel aufgetragen werden und nach dem Verdunsten des Lösungsmittels auf dem Applikationsort einen kontinuierlichen Film ausbilden.

Es wird eine Mischung aus nichtionischen Filmbildnern eingesetzt, wobei als nichtionischer Filmbildner ein Copolymerisat aus N-Vinylpyrrolidon- und C₁₀₋₂₀-Alkylen-Einheiten, insbesondere aus N-Vinylpyrrolidon- und Hexadecen-Einheiten, eingesetzt wird, wobei das Copolymerisat vorzugsweise ein Molekulargewicht von 1000 - 20000 g/mol, besonders bevorzugt 5000 - 10000 g/mol, aufweist. Copolymerisate aus N-Vinylpyrrolidon- und Hexadecen-Einheiten sind beispielsweise unter dem Handelsnamen Antaron V216 (ISP Global) oder Unimer U 151 (Induchem) erhältlich. Der nichtionische Filmbildner wird hierbei vorzugsweise in einer Menge von 2 - 18 Gew.-%, besonders bevorzugt in einer Menge von 5 -15 Gew.-% eingesetzt.

Erfindungsgemäß wird eine Mischung von mindestens zwei nichtionischen Filmbildnern eingesetzt, wobei eine Mischung eines Copolymerisats aus N-Vinylpyrrolidon- und C₁₀₋₂₀-Alkylen-Einheiten, insbesondere aus N-Vinylpyrrolidon- und Hexadecen-Einheiten, mit einem weiteren nichtionischen Filmbildner eingesetzt wird, wobei als weiterer nichtionischer Filmbildner ein Copolymer aus N-Vinylpyrrolidon-, Methacrylamid- und N-Vinyl-Imidazol-Einheiten eingesetzt wird, wie es beispielsweise unter dem Handelsnamen Luviset Clear (BASF) im Handel erhältlich ist. Das Copolymer aus N-Vinylpyrrolidon-, Methacrylamid- und N-Vinyl-Imidazol-Einheiten weist bevorzugt ein Molekulargewicht von 30000 bis 350000 g/mol, besonders bevorzugt 240000 bis 300000 g/mol, auf, wie es beispielsweise unter dem Handelsnamen Luviset Clear (BASF) erhältlich ist. Das Copolymerisat aus N-Vinylpyrrolidon- und C₁₀₋₂₀-Alkylen-Einheiten wird hierbei vorzugsweise in einer Menge von 2 - 18 Gew.-%, besonders bevorzugt in einer Menge von 5 - 15 Gew.-%, insbesondere 8 - 12 Gew.-%, und das Copolymer aus N-Vinylpyrrolidon-, Methacrylamid- und N-Vinyl-Imidazol-Einheiten vorzugsweise in einer Menge von 1 - 6 Gew.-%, besonders bevorzugt in einer Menge von 4 - 6 Gew.-%, eingesetzt.

Die Benefit-Substanz ist ausgewählt aus UV-Filtersubstanzen und/oder Bräunungsmitteln oder Mischungen davon, insbesondere Mischungen von UV-Filtersubstanzen und/oder Bräunungsmitteln mit Feuchthaltemitteln (Moisturizer). Die Benefit-Substanz ist in den erfindungsgemäßen Mitteln in einer Menge von 0,1 - 20 Gew.-%, insbesondere 0,2 -15 Gew.-%, besonders bevorzugt 0,5 -10 Gew.-% enthalten.

Bei den UV-Filtersubstanzen handelt es sich um organische oder anorganische bei Raumtemperatur flüssig oder kristallin vorliegende Substanzen, die, in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Im Folgenden werden erfindungsgemäß bevorzugte UV-Filtersubstanzen aufgeführt.

Erfindungsgemäß bevorzugte Alkyl- und/oder Alkoxy-substituierte Dibenzoylmethanderivate sind dadurch gekennzeichnet, dass einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als PARSOL^{®} 1789 von DSM oder als Eusolex^{®} 9020 von Merck KGaA erhältlich ist.

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

Bevorzugt eingesetzte Derivate der Benzimidazolsulfonsäure sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte Derivate der Benzimidazolsulfonsäure sind die 2-Phenylbenzimidazol-5-sulfonsäure (UV-B) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere aber die 2-Phenylbenzimidazol-5-sulfonsäure selbst mit der INCI-Bezeichnung "Phenylbenzimidazole sulfonic acid" (CAS.-Nr.

27503-81-7), die beispielsweise unter den Handelsnamen Neo Heliopan Hydro von Symrise oder Eusolex 232 von Merck KGaA erhältlich ist.

In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist.

In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.

UV-Filtersubstanzen auf Basis von 1,3,5-Triazinderivaten sind im Stand der Technik bekannt, beispielsweise aus EP 775698, EP 878469 und EP 1027881.

Hinsichtlich der C₃-Achse des Triazin-Grundkörpers dieser Verbindungen sind sowohl symmetrische Substitution als auch unsymmetrische Substitution denkbar. In diesem Sinne symmetrisch substituierte s-Triazine weisen in den Positionen 2, 4 und 6 drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.

Hinsichtlich der C₃-Achse des Triazin-Grundkörpers symmetrische Triazinderivate sind bevorzugt solche mit R¹ = R² = R³ = -NH-Phe-COOR, mit Phe = Phenyl-Rest, bei dem die Substituenten -NH und -COOR in para-Position zueinander stehen. Besonders bevorzugte derartige symmetrische Triazinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist.

Erfindungsgemäß bevorzugte unsymmetrische Triazinderivate sind beispielsweise solche, die in EP 775698 offenbart sind:

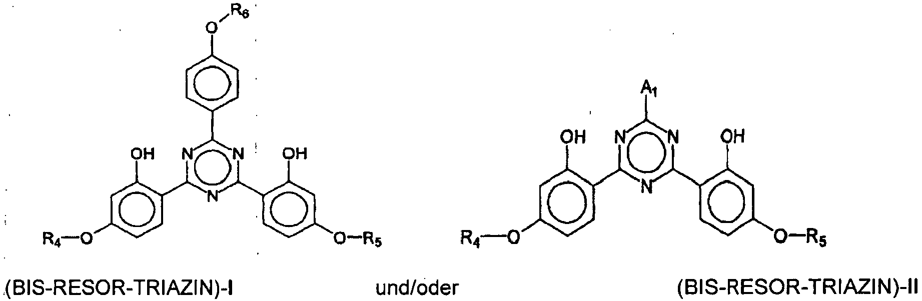

Alle in EP 775698 erwähnten so genannten Bis-Resorcinyltriazine, seien sie durch generische oder durch konkrete Formeln offenbart, sind vorteilhaft im Sinne der vorliegenden Erfindung.

Ganz besonders bevorzugt werden R₄ und R₅ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein. A₁ stellt bevorzugt einen substituierten homo- oder heterocyclischen aromatischen Fünfring oder Sechsring dar.

Ganz besonders bevorzugte Triazine sind ausgewählt aus unsymmetrisch substituierten s-TriazinVerbindungen der allgemeinen Formel (BIS-RESOR-TRIAZIN)-I, in der R₆ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-I, in der R₄ und R₅ jeweils eine 2-Ethylhexyl-Gruppe und R₆ eine Methylgruppe darstellen, ist 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere, erfindungsgemäß bevorzugte optionale unsymmetrisch substituierte s-TriazinVerbindung ist eine Verbindung mit der INCI-Bezeichnung Diethylhexyl Butamido Triazone, mit des substituierten Triazingrundkörpers mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist.

Weitere, erfindungsgemäß bevorzugte optionale UV-Filtersubstanzen auf Basis von s-TriazinVerbindungen sind: 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA; 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine); 2,4-Bis-{[4-(3-sulfonato)-2-hydroxy-propyloxy)-2-hydroxy]-phenyl}-6-(4-methoxyphenyl)-1,3,5-triazin-Natriumsalz; 2,4-Bis-{[4-(3-(2-propyloxy)-2-hydroxy-propyloxy)-2-hydroxy]-phenyl}-6-(4-methoxyphenyl)-1,3,5-triazin; 2,4-Bis-{[4-(2-ethyl-hexyloxy)-2-hydroxy]-phenyl}-6-[4-(2-methoxyethyl-carboxyl)-phenyl-amino]-1,3,5-triazin; 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin;2,4-Bis-{[4-(2-ethyl-hexyloxy)-2-hydroxy]-phenyl}-6-(1-methyl-pyrrol-2-yl)-1,3,5-triazin; 2,4-Bis-{[4-tris(trimethylsiloxy-silylpropyloxy)-2-hydroxy]-phenyl}-6-(4-methoxyphenyl)-1,3,5-triazin; 2,4-Bis-([4-(2-methylpropenyloxy)-2-hydroxy]-phenyl}-6-(4-methoxyphenyl)-1,3 ,5-triazin und 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin; 2-[4,6-Bis(2,4-dimethylphenyl)-1,3,5-triazin-2-yl]-5-(ethylhexyloxy)phenol.

Die s-Triazinderivate werden bevorzugt in die Ölphase der erfindungsgemäßen kosmetischen oder dermatologischen Zusammensetzungen eingearbeitet.

Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 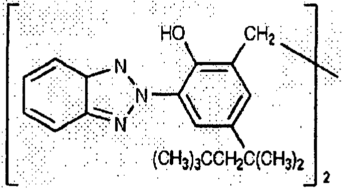 unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amyl-phenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzo-triazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte organische UV-Filtersubstanzen sind im Folgenden genannt:

Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter der Warenbezeichnung Eusolex 6300 vertrieben wird; Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich). 4-Aminobenzoësäure-Derivate, bevorzugt 4-(Dimethylamino)-benzoësäure(2-ethylhexyl) ester, 4-(Dimethylamino)benzoësäureamylester; 2-Aminobenzoesäure-Derivate; Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene));

Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat); Salicylsäurehomomenthylester (Homomenthylsalicylat, Homosalate). Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon oder 2-(4'-Diethylamino-2'-hydroxybenzoyl)-benzoesäurehexylester (auch: Aminobenzophenon, unter der Bezeichnung Uvinul A Plus bei der Firma BASF erhältlich), sowie Mischungen dieser Benzophenon-Derivate mit gegebenenfalls substituierten Estern der Zimtsäure, insbesondere mit p-Methoxyzimtsäure-2-ethylhexylester, wobei besonders bevorzugt eine Mischung aus 2-(4'-Diethylamino-2'-hydroxybenzoyl)-benzoesäurehexylester und p-Methoxyzimtsäure-2-ethylhexylester (unter der Bezeichnung Uvinul A Plus B bei der Firma BASF erhältlich) eingesetzt wird; Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze; Ester der Benzalmalonsäure, bevorzugt 4-Methoxybenzalmalonsäuredi(2-ethylhexyl)ester, sowie an Polymere gebundene UV-Filter, die von Komponente (b) verschieden sind.

Derivate von Benzoxazol, bevorzugt 2,2'(Naphthalene-1,4-Diyl)bis(benzoxazole) (INCI: Dibenzoxazoyl Naphthalene) und 2,4-bis-[5-1 (di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer erfindungsgemäß ganz besonders bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat, wobei 2-Cyano-3,3-phenylzimtsäure-2-ethylhexylester (Octocrylene) besonders bevorzugt ist. In diesen Kombinationen liegt das Gewichtsverhältnis von UV-B-Filter zu 1-(4-tert.-Butylphenyl)-3-(4'methoxyphenyl)propan-1,3-dion vorzugsweise zwischen 1:1 und 10:1, besonders bevorzugt zwischen 2:1 und 8:1, das molare Verhältnis liegt entsprechend zwischen 0,3 und 3,8, bevorzugt zwischen 0,7 und 3,0.

Die Liste der genannten optionalen UV-Filtersubstanzen, die im Sinne der vorliegenden Erfindung eingesetzt werden können, soll selbstverständlich nicht limitierend sein. Die Gesamtmenge an organischen UV-Filtersubstanzen in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung, wobei in einer bevorzugten Ausführungsform 0,5 - 10, 0 Gew.-%, besonders bevorzugt 1,0 - 6,0 Gew.-%, organische UV-A-Filter und 0,5 - 10,0 Gew.-%, besonders bevorzugt 1,0 - 8,0 Gew.-%, organische UV-B-Filter eingesetzt werden.

Bei den erfindungsgemäß bevorzugten optional einsetzbaren anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira.

In einer erfindungsgemäß bevorzugten Ausführungsform ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Als Bräunungswirkstoff können die erfindungsgemäßen Zusammensetzungen Dihydroxyaceton (Abkürzung: DHA, 1,3-Dihydroxypropan-2-on, Ketotriose, INCI-Bez.: Dihydroxyacetone, EINECS 2024945) oder Erythrulose (1,2,3,4-Trihydroxybutan-2-on) oder Mischungen davon enthalten. Erfindungsgemäß bevorzugte Zusammensetzungen enthalten 0,5 - 15,0 Gew.-%, insbesondere 1,0 - 10,0 Gew.-%, besonders bevorzugt 1,5 - 5,0 Gew.-% und vor allem 1,75 - 3 Gew.-% eines Bräunungswirkstoffs. Als Feuchhaltemittel können beispielsweise Glycerin oder quaternäre Ammoniumverbindungen eingesetzt werden. Bevorzugt können in den erfindungsgemäßen Zusammensetzungen Bräunungmittel und/oder UV-Filtersubstanzen in Kombination mit Feuchthatlemitteln eingesetzt sein.

Die erfindungsgemäßen Zusammensetzungen können Fettstoffe enthalten. Für erfindungsgemäße fettstoffhaltige Mittel wird Schutz begehrt wird oder kann Schutz begehrt werden; Fettstoffe tragen zum technischen Ziel der Erfindung und somit zur Lösung der der anmeldungsgemäßen Erfindung zugrunde liegenden technischen Aufgabe bei. Bevorzugte Fettstoffe, die Mengen, in denen sie in erfindungsgemäßen Zusammensetzungen enthalten sind sind im Prioritätsdokument DE102008032179 auf den Seiten 14 bis 18 offenbart, die dort genannten Merkmale gehören eindeutig implizit zur Beschreibung der in der eingereichten Anmeldung enthaltenen Erfindung und damit zum Offenbarungsgehalt dieser Anmeldung.

Die erfindungsgemäßen Zusammensetzungen enthalten Tenside. Die erfindungsgemäße Zusammensetzung enthält Tenside vorzugsweise in einer Menge von 0,1 - 30 Gew.-%, insbesondere in einer Menge von 0,5 - 20 Gew.-%, bevorzugt in einer Menge von 2 - 15 Gew.-%, vor allem in einer Menge von 2,5 - 10 Gew.-%. Für erfindungsgemäße tensidhaltige Mittel wird Schutz begehrt wird oder kann Schutz begehrt werden; Tenside tragen zum technischen Ziel der Erfindung und somit zur Lösung der der anmeldungsgemäßen Erfindung zugrunde liegenden technischen Aufgabe bei. Bevorzugte Tenside, die Mengen, in denen sie in erfindungsgemäßen Zusammensetzungen enthalten sind sind im Prioritätsdokument DE102008032179 auf den Seiten 18 bis 25 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer erfindungsgemäß besonders bevorzugten Ausführungsform wird hierbei als nichtionisches Tensid ein Anlagerungsprodukt von Ethylenoxid (EO)-Einheiten an einen Fettalkohol, wobei der Fettalkohol vorzugsweise zwischen 10 und 22 C-Atome, besonders bevorzugt zwischen 14 und 20 C-Atome, vor allem zwischen 16 und 18 C-Atome umfasst, und wobei vorzugsweise zwischen 10 und 24, besonders bevorzugt zwischen 16 und 22, EO-Einheiten, vor allem 18, 19, 20, 21 oder 22 EO-Einheiten, an den Fettalkohol angelagert sind, eingesetzt. Ein bevorzugtes Produkt, das aus einem C₁₆₋₁₈-Alkohol mit 20 EO-Einheiten besteht, ist beispielsweise unter den Handelsnamen Eumulgin B2 (Cognis, Deutschland) erhältlich.

In einer anderen erfindungsgemäß besonders bevorzugten Ausführungsform wird als anionisches Tensid mindestens ein Salz eines C₁₂₋₂₀-Alkylphosphats eingesetzt. Erfindungsgemäß bevorzugte Salze von C₁₂₋₂₀-Alkylphosphaten, sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolammonium- oder Glucammoniumsalz vorliegen. Bevorzugt sind die Kalium-salze der genannten Phosphorsäuremonoester. Besonders bevorzugt ist Dikaliummono-cetylphosphat. Weitere erfindungsgemäß bevorzugte Salze von C₁₂₋₂₀-Alkylphosphaten sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolammonium- oder Glucammoniumsalz vorliegen. Bevorzugt sind die Kaliumsalze der genannten Phosphorsäurediester. Besonders bevorzugt ist Kaliumdicetylphosphat. Besonders bevorzugt sind Mischungen aus Mono-C₁₂₋₂₀-Alkylphosphaten und Di-C₁₂₋₂₀-Alkylphosphaten. Außerordentlich bevorzugt sind Mischungen aus Dikaliummonocetylphosphat und Kaliumdicetylphosphat.

In einer weiteren besonders bevorzugten Ausführungsform ist zusätzlich zu dem mindestens einen Salz von C₁₂₋₂₀-Alkylphosphat, insbesondere ein Salz von Cetylphosphat, mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden, in den erfindungsgemäßen tensidhaltigen Zusammensetzungen enthalten ist. Erfindungsgemäß bevorzugte C₁₄₋₂₀-Mono- oder Diacylglyceride sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid. Weitere erfindungsgemäß bevorzugte C₁₄₋₂₀-Mono- oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride. Eine erfindungsgemäß besonders bevorzugte Zusammensetzugnen umfassen ein Gemisch aus Dikaliummonocetylphosphat und Kaliumdicetylphosphat mit gehärteten Palmölglyceriden, welches z.B. als Handelsprodukt "Emulsiphos 677660" (INCI: Potassium Cetyl Phosphate, Hydrogenated Palm Glycerides) von der Firma Symrise erhältlich ist.

Die bevorzugte Kombination, bestehend aus mindestens einem Salz von C₁₂₋₂₀-Alkylphosphat als anionischem Öl-in-Wasser-Emulgator und mindestens einem C₁₄₋₂₀-Mono- oder Diacylglycerid, ist bevorzugt in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, enthalten, jeweils bezogen auf das Gesamtgewicht der erfindungsgemäßen Zusammensetzung.

Diese Formulierungen mit einem Copolymer aus N-Vinylpyrrolidon-, Methacrylamid- und N-Vinyl-Imidazol-Einheiten und einem Copolymerisat aus N-Vinylpyrrolidon- und C₁₀₋₂₀-Alkylen-Einheiten, insbesondere aus N-Vinylpyrrolidon- und Hexadecen-Einheiten, erlauben eine gute Reinigungsleistung bei gleichzeitiger Deponierung der UV-Filtersubstanz und/oder des Bräunungsmittels, ohne ein klebriges Hautgefühl zu hinterlassen. In diesen Kombinationen können mindestens eine öllösliche und/oder mindestens eine wasserlösliche UV-Filtersubstanz eingesetzt werden. Insbesondere werden die bereits als bevorzugten beschriebenen UV-Filtersubstanzen, bevorzugt die Derivate der Benzimidazolsulfonsäure, ganz besonders Phenylen-1,4-bis-(2-benzimidazyl)-3,3'-5,5'-tetrasulfonsäure (UV-A) und/oder 2-Phenylbenzimidazol-5-sulfonsäure (UV-B) und ihre Salze und/oder die Alkyl- und/oder Alkoxy-substituierten Dibenzoylmethanderivate, ganz besonders bevorzugt 4-tert-Butyl-4'-Methoxydibenzoylmethan, eingearbeitet.

Ganz besonders bevorzugt sind die Kombinationen von Kaliumdicetylphosphat (z.B. auch in einem Gemisch aus Dikaliummonocetylphosphat und Kaliumdicetylphosphat mit gehärteten Palmölglyceriden), Phenylen-1,4-bis-(2-benzimidazyl)-3,3'-5,5'-tetrasulfonsäure-bis-natriumsalz und Butyl Methoxydibenzoylmethane mit einem N-Copolymer aus N-Vinylpyrrolidon-, Methacrylamid- und N-Vinyl-Imidazol-Einheiten und einem Copolymerisat aus N-Vinylpyrrolidon- und Hexadecen-Einheiten. Weiterhin bevorzugt sind die Kombinationen von Kaliumdicetylphosphat (z.B. auch in einem Gemisch aus Dikaliummonocetylphosphat und Kaliumdicetylphosphat mit gehärteten Palmölglyceriden), 2-Phenylbenzimidazol-5-sulfonsäure und Butyl Methoxydibenzoylmethane mit einem N-Copolymer aus N-Vinylpyrrolidon-, Methacrylamid- und N-Vinyl-Imidazol-Einheiten und einem Copolymerisat aus N-Vinylpyrrolidon- und Hexadecen-Einheiten. Diese Kombinationen können zusätzlich auch Polyester-5 enthalten.

Eine erfindungsgemäße Zusammensetzung kann weiterhin mindestens einen Pflanzenextrakt enthalten. Für erfindungsgemäße pflanzenextrakthaltige Mittel wird Schutz begehrt wird oder kann Schutz begehrt werden; Pflanzenextrakt tragen zum technischen Ziel der Erfindung und somit zur Lösung der der anmeldungsgemäßen Erfindung zugrunde liegenden technischen Aufgabe bei. Bevorzugte Pflanzenextrakt, die Mengen, in denen sie in erfindungsgemäßen Zusammensetzungen enthalten sind sind im Prioritätsdokument DE102008032179 auf den Seiten 25 bis 27 offenbart, die dort genannten Merkmale gehören eindeutig implizit zur Beschreibung der in der eingereichten Anmeldung enthaltenen Erfindung und damit zum Offenbarungsgehalt dieser Anmeldung.

Neben den erfindungsgemäß einzusetzenden polymeren Filmbildnern kann eine erfindungsgemäße Zusammensetzung auch weitere Polymere enthalten. Für erfindungsgemäße tensidhaltige Mittel wird Schutz begehrt wird oder kann Schutz begehrt werden; Polymere tragen zum technischen Ziel der Erfindung und somit zur Lösung der der anmeldungsgemäßen Erfindung zugrunde liegenden technischen Aufgabe bei. Bevorzugte Polymere, die Mengen, in denen sie in erfindungsgemäßen Zusammensetzungen enthalten sind sind im Prioritätsdokument DE102008032179 auf den Seiten 27 bis 34 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren Ausführungsform der erfindungsgemäßen Mittel kann die Wirkung durch die Verwendung von Proteinhydrolysaten und deren Derivaten und/oder Monomeren, Oligomeren und Polymeren von Aminosäuren und N-C₂-C₂₄-Acylaminosäuren sowie Ester und/oder physiologisch verträgliche Metallsalze dieser Substanzen gesteigert werden. Für die erfindungsgemäßen Mittel, welche die Proteinhydrolysate und deren Derivate, und/oder Monomeren, Oligomeren und Polymeren von Aminosäuren und N-C₂-C₂₄-Acylaminosäuren sowie Ester und/oder physiologisch verträgliche Metallsalze dieser Substanzen, ggf. auch in geträgerter Form, enthalten, wird Schutz begehrt wird oder kann Schutz begehrt werden; Sie tragen zum technischen Ziel der Erfindung und somit zur Lösung der der anmeldungsgemäßen Erfindung zugrunde liegenden technischen Aufgabe bei. Bevorzugte Proteinhydrolysate und deren Derivate, und/oder Monomeren, Oligomeren und Polymeren von Aminosäuren und N-C₂-C₂₄-Acylaminosäuren sowie Ester und/oder physiologisch verträgliche Metallsalze dieser Substanzen, die Mengen, in denen sie in erfindungsgemäßen Zusammensetzungen enthalten sind sind im Prioritätsdokument DE102008032179 auf den Seiten 35 bis 39 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform sind die Polymeren der Aminosäuren ausgewählt aus DNA-Reparaturenzymen. Erfindungsgemäß bevorzugte DNA-Reparaturenzyme sind Photolyse und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus Anacystis nidulans, einem phototrophen marinen Mikroorganismus. Die Photolyase aus A. nidulans wird in technisch relevanten Mengen mittlerweile aus E. coli gewonnen. Photolyase ist zur Aktivierung auf Licht angewiesen. Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv. Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich. In den erfindungsgemäßen Zusammensetzungen sind die Photosome™ oder Ultrasome™ in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.

Die erfindungsgemäßen Zusammensetzungen können weiterhin Betain-Verbindungen enthalten.

Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.

Die Betainverbindungen sind in den erfindungsgemäßen Mitteln, sofern eingesetzt, vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

Weiterhin können die erfindungsgemäßen Zusammensetzungen neben den hydrophob modifizierten Polysacchariden weitere Zuckerbestandteile ausgewählt aus Mono-, Oligo- und/oder Polysacchariden und/oder deren Derivaten enthalten. Für erfindungsgemäße zuckerbestandteilhaltige Mittel wird Schutz begehrt wird oder kann Schutz begehrt werden; Zuckerbestandteile tragen zum technischen Ziel der Erfindung und somit zur Lösung der oder anmeldungsgemäßen Erfindung zugrunde liegenden technischen Aufgabe bei. Bevorzugte Zuckerbestandteile, die Mengen, in denen sie in erfindungsgemäßen Zusammensetzungen enthalten sind sind im Prioritätsdokument DE102008032179 auf den Seiten 39 bis 40 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform enthalten die erfindungsgemäßen Zusammensetzungen mindestens eine α-Hydroxycarbonsäure, α-Ketocarbonsäure oder β-Hydroxycarbonsäure oder deren Ester-, Lacton- oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind, sofern eingesetzt, vorzugsweise in Mengen von 0,1 - 10 Gew.-%, bevorzugt 0,5 - 5 Gew.-%, jeweils bezogen auf die gesamte Zusammensetzung, enthalten.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol. In einer erfindungsgemäß bevorzugten Ausführungsform wird Glycerin in einer Menge von 2 bis 5 Gew.-% eingesetzt.

Als Konservierungsmittel eignen sich beispielsweise Phenoxyethanol, Formaldehydlösung, Parabene, insbesondere Methyl- oder Propylparaben, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage. In einer weiteren bevorzugten Ausführungsform enthalten die erfindungsgemäßen Zusammensetzungen mindestens ein Vitamin, Provitamin oder eine als Vitaminvorstufe (im weiteren Vitamine) bezeichnete Verbindung aus den Vitamingruppen A, B, C, E, H und K und den Estern der vorgenannten Substanzen. Die erfindungsgemäßen Zusammensetzungen können Vitamine enthalten. Für erfindungsgemäße tensidhaltige Mittel wird Schutz begehrt wird oder kann Schutz begehrt werden; Vitamine tragen zum technischen Ziel der Erfindung und somit zur Lösung der der anmeldungsgemäßen Erfindung zugrunde liegenden technischen Aufgabe bei. Bevorzugte Vitamine, die Mengen, in denen sie in erfindungsgemäßen Zusammensetzungen enthalten sind sind im Prioritätsdokument DE102008032179 auf den Seiten 42 bis 43 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform enthalten die erfindungsgemäßen Zusammensetzungen mindestens ein Polyphenol, Flavonoid und/oder Isoflavonoid (oder mindestens einen Polyphenol-, Flavonoid- und/oder Isoflavonoidreichen Pflanzenextrakt). Für erfindungsgemäße polyphenol-, flavonoid- bzw. isoflavonoidhaltige Mittel wird Schutz begehrt wird oder kann Schutz begehrt werden; Polyphenole, Flavonoide und/oder Isoflavonoide tragen zum technischen Ziel der Erfindung und somit zur Lösung der der anmeldungsgemäßen Erfindung zugrunde liegenden technischen Aufgabe bei. Bevorzugte Polyphenole, Flavonoide bzw. Isoflavonoide, die Mengen, in denen sie in erfindungsgemäßen Zusammensetzungen enthalten sind sind im Prioritätsdokument DE102008032179 auf den Seiten 43 bis 45 offenbart, die dort genannten Merkmale gehören eindeutig implizit zur Beschreibung der in der eingereichten Anmeldung enthaltenen Erfindung und damit zum Offenbarungsgehalt dieser Anmeldung.

Die erfindungsgemäßen Zusammensetzungen können ferner ein Ubichinon oder ein Ubichinol oder deren Derivate (im weiteren Ubichinol-Verbindungen), einen sebumregulierenden Wirkstoff und/oder Antioxidantien enthalten. Für diese erfindungsgemäßen Ubichinol-Verbindungshaltigen, Sebumregulatorhaltigen und/oder antioxidantienhaltige Mittel wird Schutz begehrt wird oder kann Schutz begehrt werden; Sebumregulatoren, Ubichinol-Verbindung und/oder Antioxidantien tragen zum technischen Ziel der Erfindung und somit zur Lösung der der anmeldungsgemäßen Erfindung zugrunde liegenden technischen Aufgabe bei. Bevorzugte Sebumregulatoren, Ubichinol-Verbindung und/oder Antioxidantien, die Mengen, in denen sie in erfindungsgemäßen Zusammensetzungen enthalten sind sind im Prioritätsdokument DE102008032179 auf den Seiten 45 bis 47 offenbart, die dort genannten Merkmale gehören eindeutig implizit zur Beschreibung der in der eingereichten Anmeldung enthaltenen Erfindung und damit zum Offenbarungsgehalt dieser Anmeldung.

Weitere Wirk-, Hilfs- und Zusatzstoffe sind beispielsweise:
Weitere Verdickungsmittel außer den bereits genannten Polysacchariden wie Gelatine oder Pflanzengumme, beispielsweise Agar-Agar, Guar-Gum; Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen; Strukturanten wie Maleinsäure und Milchsäure; Parfümöle; Dimethylisosorbid; Cyclodextrine; Lösungsmittel und -vermittler wie Ethanol, Isopropanol, Ethylenglykol, Propylenglykol, Glycerin und Diethylenglykol; faserstruktur-verbessernde Wirkstoffe, insbesondere Mono-, Di- und Oligosaccharide wie beispielsweise Glucose, Galactose, Fructose, Fruchtzucker und Lactose; quaternierte Amine wie Methyl-1-alkylamidoethyl-2-alkylimidazolinium-methosulfat; Entschäumer wie Silikone; Farbstoffe zum Anfärben des Mittels; Antischuppenwirkstoffe wie Piroctone Olamine, Zink Omadine und Climbazol; weitere Substanzen zur Einstellung des pH-Wertes, wie beispielsweise α- und β-Hydroxycarbonsäuren; Wirkstoffe wie Allantoin und Bisabolol; Cholesterin; Komplexbildner wie EDTA, NTA, β-Alanindiessigsäure und Phosphonsäuren; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Ceramide. Unter Ceramiden werden N-Acylsphingosin (Fettsäureamide des Sphingosins) oder synthetische Analogen solcher Lipide (sogenannte Pseudo-Ceramide) verstanden; Trübungsmittel wie Latex, Styrol/PVP- und Styrol/Acrylamid-Copolymere; Perlglanzmittel wie Ethylenglykolmono- und - distearat sowie PEG-3-distearat; Pigmente; Reduktionsmittel wie z. B. Thioglykolsäure und deren Derivate, Thiomilchsäure, Cysteamin, Thioäpfelsäure und α-Mercaptoethansulfonsäure; Treibmittel wie Propan-Butan-Gemische, N₂O, Dimethylether, CO₂ und Luft; Antioxidantien; Desoxyzucker; Pflanzenglycoside; Polysaccharide wie Fucose oder Rhamnose.

Bezüglich weiterer fakultativer Komponenten sowie die eingesetzten Mengen dieser Komponenten wird ausdrücklich auf die dem Fachmann bekannten einschlägigen Handbücher, z. B. die Monographie von Kh. Schrader, Grundlagen und Rezepturen der Kosmetika, 2. Auflage, Hüthig Buch Verlag, Heidelberg, 1989, verwiesen.

Als Konfektionierung erfindungsgemäßer Zusammensetzungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen, Gele, Sprays, Aerosole und Schaumaerosole geeignet. Diese werden in der Regel auf wäßriger oder wäßrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wäßrig alkoholischen Basis in einem Gewichtsverhältnis von 1:10 bis 10:1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Monoethanolamin, Triethanolamin sowie N,N,N',N'-Tetrakis-(2-hydroxypropyl)-ethylendiamin.

Bei den erfindungsgemäßen Zusammensetzungen handelt es sich vorzugsweise um Rinse-Off-Produkte, insbesondere um reinigende Mittel für Haut und/oder Haar. Es kann sich hierbei insbesondere um ein Waschgel, eine Waschcreme, ein Waschmousse, ein Gesichtswasser, eine Reinigungsmilch, eine Reinigungslotion, ein Reinigungstonic, eine Gesichtsmaske, eine Haarkur, eine Haarspülung, ein Haarconditioner, ein Shampoo, ein Duschgel, ein Duschöl, ein Duschmousse, einen Duschcreme, eine Seife oder einen Make-up-Entferner handeln.

In einer besonderen Ausführungsform der erfindungsgemäßen Mittel kann es bevorzugt sein, wenn die Mittel als Mikroemulsion vorliegen. Unter Mikroemulsionen werden im Rahmen der Erfindung ebenfalls sogenannte "PIT"-Emulsionen verstanden. Einzelheiten bezüglich dieser sehr stabilen, niedrigviskosen Systeme, für die sich die Bezeichnung "PIT-Emulsionen" allgemein durchgesetzt hat, sind einer Vielzahl von Druckschriften zu entnehmen, für die stellvertretend die Veröffentlichungen in Angew. Chem. 97, 655-669 (1985) und Adv. Colloid Interface Sci 58, 119-149 (1995) genannt werden. Erfindungsgemäß können solche Mikro- oder "PIT"-Emulsionen bevorzugt sein, die einen mittleren Teilchendurchmesser von etwa 200 nm aufweisen.

Die Herstellung der erfindungsgemäßen Mikroemulsionen kann beispielsweise in der im Prioritätsdokument DE102008032179 auf den Seite 49 bis 50 beschriebenen Art erfolgen. Bezüglich bevorzugter Verwendungen gilt mutatis mutandis das zu den erfindungsgemäßen tensidhaltigen Mitteln Ausgeführte.

### Ausführungsbeispiele (Mengenangaben alle in Gew.-%)

Beispiel 1: Eine Mischung der UV-Filter Parsol 1789 (Butyl Methoxydibenzoylmethane) (UVA) und Uvinul N 539 T (Octocrylene) (UVB) wurde in die Grundrezeptur eines Gesichtsreinigers eingearbeitet (Tabelle 1), wobei in Rezeptur 2 zusätzlich eine Mischung aus den zwei nichtionischen Filmbildnern Antaron V216 (ein Copolymer aus N-Vinylpyrrolidon- und Hexadecen-Einheiten) und Luviset Clear (ein Copolymer aus N-Vinylpyrrolidon-, Methacrylamid- und N-Vinyl-Imidazol-Einheiten) anwesend war. Der Gesichtsreiniger wurde auf ein Hautmodell von VitroSkin aufgetragen und dort für 3 Minuten bei 25°C belassen. Danach wurde der Gesichtsreiniger mit Wasser abgewaschen. Die ermittelten UVA- (λ = 358 nm) und UVB- (λ = 302 nm) Absorptionswerte sowie der Sonnenschutzfaktor (Sun Protection Factor, SPF) sind in Tabelle 4 angegeben. Wie Tabelle 1 zu entnehmen, konnte ohne Zusatz von Filmbildner kein signifikanter Sonnenschutz erreicht werden, während die Zugabe der Mischung aus Luviset Clear und Antaron V216 zu einer deutlichen Erhöhung des Sonnenschutzfaktors führte.

**Tab. 1: Gesichtsreiniger-Formulierungen**

| | | |
|---|---|---|
| Komponente (Gew.-%)/Probennummer | 1 | 2 |
| Dibutyl Adipate | 30 | 30 |
| Ceteareth-20 | 3 | 3 |
| Antaron V216 | 0 | 10 |
| Luviset Clear | 0 | 5 |
| Octocrylene | 5 | 5 |
| Butyl Methoxydibenzoylmethane | 3 | 3 |
| Farbstofflsg. Patentblau 85E131 | 0,6 | 0,6 |
| *Absorption UVA* | 0,1 | 2,7 |
| *Absorption UVB* | 0,08 | 2,0 |

## Patentansprüche

1. Tensidhaltige Zusammensetzung enthaltend:
a) 2 - 30 Gew.-% Filmbildner,
b) 0,1 - 20 Gew.-% mindestens einer Benefit-Substanz ausgewählt aus der Gruppe bestehend aus UV-Filtersubstanzen und Bräunungsmitteln,
wobei ein nichtionischer Filmbildner, der ein Copolymer aus N-Vinylpyrrolidon- und C₁₀₋₂₀-Alkylen-Einheiten ist, und ein nichtionischer Filmbildner, der ein Copolymer aus N-Vinylpyrrolidon-, Methacrylamid- und N-Vinyl-Imidazol-Einheiten ist, enthalten sind.

2. Tensidhaltige Zusammensetzung nach Anspruch 1, **dadurch gekennzeichnet, dass** es sich bei den UV-Filtersubstanzen um eine Mischung von UV-A- und UV-B-Absorbern handelt.

3. Tensidhaltige Zusammensetzung nach einem der Ansprüche 1 bis 2, **dadurch gekennzeichnet, dass** das Copolymer aus N-Vinylpyrrolidon- und C₁₀₋₂₀-Alkylen-Einheiten ein Molekulargewicht von 1000 - 20000g/mol, bevorzugt 5000 - 10000 g/mol aufweist.

4. Tensidhaltige Zusammensetzung nach einem der Ansprüche 1 bis 3, **dadurch gekennzeichnet, dass** das Copolymer aus N-Vinylpyrrolidon- und C₁₀₋₂₀-Alkylen-Einheiten in einer Menge von 2 -18 Gew.-% enthalten ist.

5. Tensidhaltige Zusammensetzung nach einem der Ansprüche 1 bis 4, **dadurch gekennzeichnet, dass** das Copolymer aus N-Vinylpyrrolidon-, Methacrylamid- und N-VinylImidazol-Einheiten ein Molekulargewicht von 30000 bis 350000 g/mol, bevorzugt 240000 bis 300000 g/mol, aufweist.

6. Tensidhaltige Zusammensetzung nach einem der Ansprüche 1 bis 5, **dadurch gekennzeichnet, dass** das Copolymer aus N-Vinylpyrrolidon-, Methacrylamid- und N-Vinyl-Imidazol-Einheiten in einer Menge von 1 - 6 Gew.-% enthalten ist.

7. Tensidhaltige Zusammensetzung nach einem der Ansprüche 1 bis 6, **dadurch gekennzeichnet, dass** sie mindestens einen Fettstoff, insbesondere ein polares oder unpolares Öl, in einer Menge von 0,1 - 50 Gew.-% enthält.

8. Tensidhaltige Zusammensetzung nach einem der Ansprüche 1 bis 7, **dadurch gekennzeichnet, dass** sie Tenside, bevorzugt anionische oder nichtionische oder amphotere Tenside oder Mischungen davon, in einer Menge von 0,1 - 30 Gew.-% enthält.

9. Tensidhaltige Zusammensetzung nach Anspruch 8, **dadurch gekennzeichnet, dass** es sich bei dem nichtionischen Tensid um ein Anlagerungsprodukt von zwischen 16 und 22 Ethylenoxid (EO)-Einheiten an einen Fettalkohol mit zwischen 14 und 20 C-Atomen handelt.

10. Tensidhaltige Zusammensetzung nach Anspruch 8, **dadurch gekennzeichnet, dass** es sich bei dem anionischen Tensid um ein Salz eines C₁₂₋₂₀-Alkylphosphats, insbesondere ein Salz von Cetylphosphat handelt.

11. Nicht-therapeutische Verwendung einer Tensid-haltigen Zusammensetzung gemäß einem der Ansprüche 1 - 10 zur Reinigung der Haut und gleichzeitigen Verleihung von Sonnenschutz.

## Claims

1. Surfactant-containing composition comprising:
a) 2 - 30 wt % film-formers,
b) 0.1 - 20 wt % of at least one beneficial substance selected from the group consisting of UV filter substances and tanning agents,
wherein a non-ionic film-former that is a copolymer of *N*-vinyl pyrrolidone units and C₁₀₋₂₀ alkylene units, and a non-ionic film-former that is a copolymer of *N*-vinyl pyrrolidone units, methacrylamide units and *N*-vinyl imidazole units, are comprised.

2. Surfactant-containing composition according to claim 1, **characterised in that** the UV filter substances concern a mixture of UV-A and UV-B absorbers.

3. Surfactant-containing composition according to one of claims 1 to 2, **characterised in that** the molecular weight of the copolymer of *N*-vinyl pyrrolidone units and C₁₀₋₂₀ alkylene units is from 1000 - 20 000 g/mol, preferably 5000 - 10 000 g/mol.

4. Surfactant-containing composition according to one of claims 1 to 3, **characterised in that** the copolymer of *N*-vinyl pyrrolidone units and C₁₀₋₂₀ alkylene units is comprised in an amount of 2 - 18 wt %.

5. Surfactant-containing composition according to one of claims 1 to 4, **characterised in that** the molecular weight of the copolymer of *N*-vinyl pyrrolidone units, methacrylamide units and *N*-vinyl imidazole units is from 30 000 - 350 000 g/mol, preferably 240 000 - 300 000 g/mol.

6. Surfactant-containing composition according to one of claims 1 to 5, **characterised in that** the copolymer of *N*-vinyl pyrrolidone units, methacrylamide units and *N*-vinyl imidazole units is comprised in an amount of 1 - 6 wt %.

7. Surfactant-containing composition according to one of claims 1 to 6, **characterised in that** it comprises at least one fatty substance, in particular a polar or non-polar oil, in an amount of 0.1 - 50 wt %.

8. Surfactant-containing composition according to one of claims 1 to 7, **characterised in that** it comprises surfactants, preferably anionic or non-ionic or amphoteric surfactants or mixtures thereof, in an amount of 0.1 - 30 wt %.

9. Surfactant-containing composition according to claim 8, **characterised in that** the non-ionic surfactant concerns an addition product of between 16 and 22 ethylene oxide (EO) units onto a fatty alcohol containing between 14 and 20 carbon atoms.

10. Surfactant-containing composition according to claim 8, **characterised in that** the anionic surfactant concerns a salt of a C₁₂₋₂₀ alkyl phosphate, in particular a salt of cetyl phosphate.

11. Non-therapeutic use of a surfactant-containing composition according to one of claims 1 - 10 for cleansing the skin and simultaneously lending protection against the sun.

## Revendications

1. Composition comprenant un ou plusieurs agents tensioactifs contenant :
a) de 2 à 30 % en poids d' agents filmogènes
b) de 0,1 à 20 % en poids d'au moins une substance procurant un avantage, choisie parmi le groupe constitué par des substances faisant office de filtre pour les rayons ultraviolets et des agents de bronzage;
dans laquelle sont contenus, un agent filmogène non ionique qui représente un copolymère d'unités de N-vinylpyrrolidone et d'alkylène en C₁₀-C₂₀, et un agent filmogène non ionique qui représente un copolymère d'unités de N-vinylpyrrolidone, de méthacrylamide et de N-vinylimidazole.

2. Composition comprenant un ou plusieurs agents tensioactifs selon la revendication 1, **caractérisée en ce qu'**il s'agit, en ce qui concerne les substances faisant office de filtre pour les rayons ultraviolets, d'un mélange d'absorbants UV-A et UV-B.

3. Composition comprenant un ou plusieurs agents tensioactifs selon la revendication 1 ou 2, **caractérisée en ce que** le copolymère d'unités de N-vinylpyrrolidone et d'alkylène en C₁₀-C₂₀ présente un poids moléculaire de 1000 à 20.000 g/mol, de préférence de 5000 à 10.000 g/mol.

4. Composition comprenant un ou plusieurs agents tensioactifs selon l'une quelconque des revendications 1 à 3, **caractérisée en ce qu'**elle contient le copolymère d'unités de N-vinylpyrrolidone et d'alkylène en C₁₀-C₂₀ en une quantité de 2 à 18 % en poids.

5. Composition comprenant un ou plusieurs agents tensioactifs selon l'une quelconque des revendications 1 à 4, **caractérisée en ce que** le copolymère d'unités de N-vinylpyrrolidone, de méthacrylamide et de N-vinylimidazole présente un poids moléculaire de 30.000 à 350.000 g/mol, de préférence de 240.000 à 300.000 g/mol.

6. Composition comprenant un ou plusieurs agents tensioactifs selon l'une quelconque des revendications 1 à 5, **caractérisée en ce qu'**elle contient le copolymère d'unités de N-vinylpyrrolidone, de méthacrylamide et de N-vinylimidazole en une quantité de 1 à 6 % en poids.

7. Composition comprenant un ou plusieurs agents tensioactifs selon l'une quelconque des revendications 1 à 6, **caractérisée en ce qu'**elle contient au moins une substance grasse en particulier une huile polaire ou apolaire en une quantité de 0,1 à 50 % en poids.

8. Composition comprenant un ou plusieurs agents tensioactifs selon l'une quelconque des revendications 1 à 7, **caractérisée en ce qu'**elle contient des agents tensioactifs, de préférence des agents tensioactifs anioniques ou non ioniques ou amphotères ou leurs mélanges en une quantité de 0,1 à 30 % en poids.

9. Composition comprenant un ou plusieurs agents tensioactifs selon la revendication 8, **caractérisée en ce qu'**il s'agit, en ce qui concerne l'agent tensioactif non ionique d'un produit de fixation par addition de 16 à 22 unités d'oxyde d'éthylène (EO) à un alcool gras contenant entre 14 et 20 atomes de carbone.

10. Composition comprenant un ou plusieurs agents tensioactifs selon la revendication 8, **caractérisée en ce qu'**il s'agit, en ce qui concerne l'agent tensioactif anionique d'un sel d'un phosphate d'alkyle en C₁₂-C₂₀, en particulier d'un sel de phosphate de cétyle.

11. Utilisation non thérapeutique d'une composition comprenant un ou plusieurs agents tensioactifs selon l'une quelconque des revendications 1 à 10 pour le nettoyage de la peau et la protection simultanée contre le soleil.
